(19) Europäisches Patentamt
European Patent Office
Office européen des brevets

(11) Publication number : **0 496 474 A2**

(12) # EUROPEAN PATENT APPLICATION

(21) Application number : **92200164.9**

(22) Date of filing : **21.01.92**

(51) Int. Cl.[5] : **C07F 9/117,** A61K 31/66, C07C 229/26, C07C 279/14, C07D 295/10

PRIORITY CLAIMED: ITALY MI91A000133,DATED: 22-01-1991.

(30) Priority : **22.01.91**

(43) Date of publication of application : **29.07.92 Bulletin 92/31**

(84) Designated Contracting States : **AT BE CH DE DK ES FR GB GR IT LI LU NL PT SE**

(71) Applicant : **GOLGI S.A.**
**Via Giulia 43**
**CH-6855 Stabio (CH)**

(72) Inventor : **Previtali, Emanuela**
**Via Manzoni, 3**
**IT-22050 Abbadia Lariana (Como) (IT)**

(74) Representative : **Dragotti, Gianfranco et al**
**SAIC BREVETTI s.r.l. Viale Bianca Maria, 15**
**I-20122 Milano (IT)**

(54) **Saline derivates of glyceryl phosphoryl myoinositol having therapeutical activity, their preparation and pharmaceutical compositions containing them.**

(57) The saline derivatives of glyceryl phosphoryl myoinositol with organic bases or pharmacologically active basic organic compounds, having the formula :

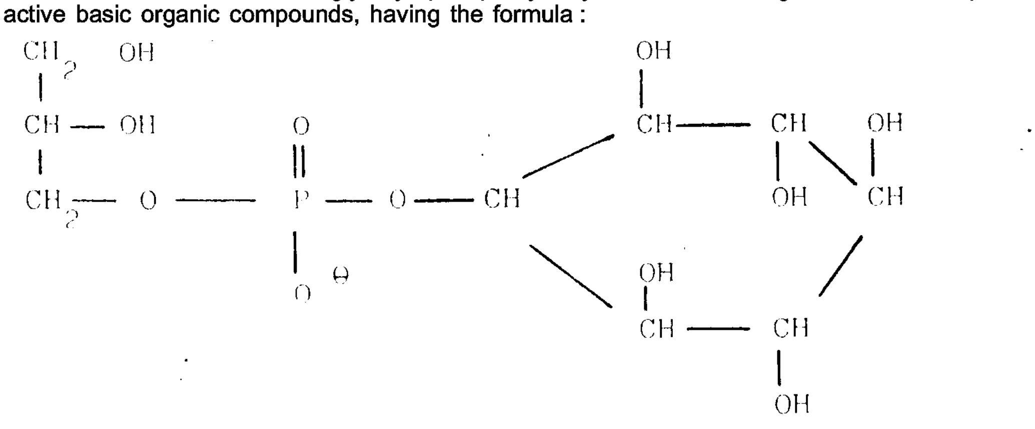

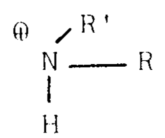

are therapeutically useful in the treatment of cerebral syndromes related to the ageeing.

EP 0 496 474 A2

The present invention relates to novel chemical compounds obtained from the salification of glyceryl phosphoryl myoinositol with organic bases consisting of basic aminoacids or of basic organic compounds pharmacologically active in a synergic manner so as to complete the pharmocological activity of the glyceryl phosphoryl myoinositol as such, having the following formula:

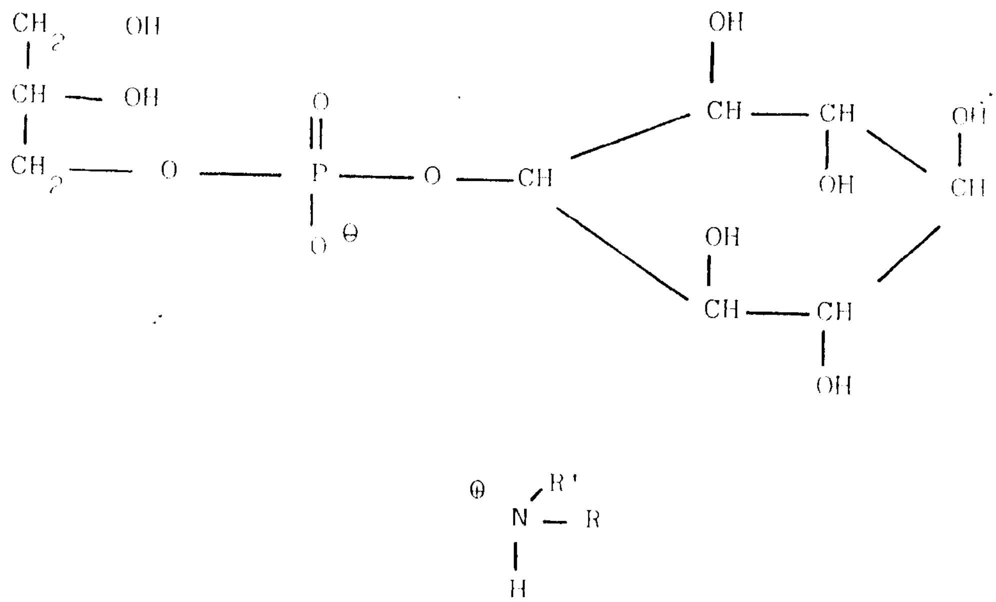

wherein:
when R represents the residue of a basic aminoacid R′ = H, and
when R represents the group of a cyclic ring having 4 or 5 carbon atoms (pyrrolidine or piperidine group), R′ represents a simple or variously substituted aliphatic chain.

The glyceryl phosphoryl myoinositol is mainly used at the cerebral level and is generally suggested in the ageeing period for the use in long run therapies with rather high daily dosages.

For this reason it is preferable to avoid the use of salts of glyceryl phosphoryl myoinositol with inorganic ions like sodium, potassium, calcium, since the electrolitic situation of the patients might be affected, causing side drawbacks not consistent with the general clinical picture.It has been thus considered as suitable to prepare some organic salts of such an active principle using basic aminoacides,such as L and DL lysine, arginine, ornithine, having an inert behaviour as regards the main activity of glyceryl phosphoryl mioinositol, but capable of avoiding the possible side effects related to the inorganic salts.

It has been furthermore considered it important to evoluate also some salts with organic bases having a mild synergic activity in order to complete a clinical situation often occuring when problems arise related to the circulatory apparatus. It has been thus prepared the salt with 4 (1-pyrrolidinyl) 1 (2,4,6-trimethoxyphenyl) 1-butanone which according to the tests is endowed not only with a vasodilating action but also with a specific platelet antiaggregating action.

From the above information it is evident that the above defined organic salts of glyceryl phosphoryl myoinositol can be advantageously used for the treatment of cerebral syndromes of different nature, in form of pharmaceutical compositions orally and parenterally administerable.

For the formulation of the pharmaceutical compositions containing the compounds of the invention recourse is made to the standard pharmaceutical technique by using excipients of conventional nature as well as to the possibility of combining therewith other active principles having complementary action or anyhow useful for the foreseen therapeutical use.

Examples of pharmaceutical formulations suitable for oral administration comprise capsules, soft capsules, tablets, granulates, powders, solutions, lyophilized tiny bottles, sachets, as well as possible delayed release formulations, etc. containing 20 to 800 mg of active compound for dosage unit, to be administered from 1 to 3 times daily depending on the diagnosis and on the patient conditions.

A number of examples are hereinafter given for illustrative purpose, showing the several salificationsz without however having limiting purpose.

The aqueous solutions of glyceryl phosphoryl myoinositol used for the preparation of the salts of the present invention were obtained by treating with a cationic resin in acid form the corresponding cyclohexylammonium

salt having the formula (1) (Brown, J.Chem.Soc.,3774 (1961)).

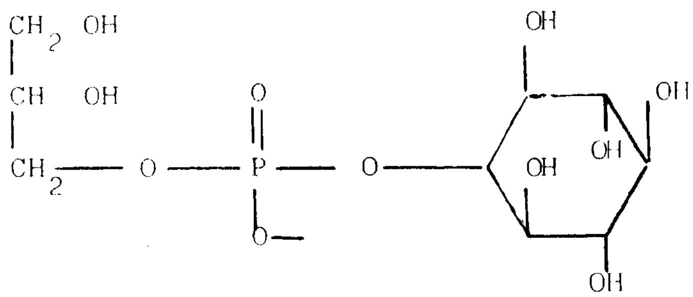

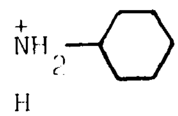

EXAMPLE 1

Glyceryl phosphoryl myoinositol L-arginine salt

5.266 g (corresponding to 0.0274 moles) of dihydrated arginine (base) are carefully weighed and added under mechanical stirring to an aqueous solution containing 9.152 g (0.274 moles) of glyceryl phosphoryl myoinositol. After the addition the final pH must be 4.6/4.7 representing the pH of the final salt.This solution is then poured in a flask and concentrated to dryness under vacuum. It is taken with hot ethanol and then slowly crystallized until fine white crystals are formed after stirring for 12 hours at +5°C. There are obtained 9 g of final product having m.p. 106/107°C.

HPLC titration :

93.87% (on dry basis)

82.6% (on the obtained product as such).

$[\alpha]^{20}_d$ (calculated on dry basis) : -0.7° (c=5 $H_2O$).

EXAMPLE 2

Glyceryl phosphoryl myoinosiltol DL-lysine salt

An amount of DL-lysin as a 50% aqueous solution is weighed corresponding to 0.0126 moles as well as to 3.7 g of 50% solution.

This aqueous solution is poured ia an aqueous solution of 4.224 g (0.0126 moles) of glyceryl phosphoryl myoinositol.At the end of the addition the final solution of the salt has a pH of between 4.7 and 4.8. This solution is poured in a flask and concentrated under vacuum in a pulsor. The product is taken with ethanol,and again dissolved and concentrated.

The concentrated solution is poured in 200 ml of anhydrous ethanol under stirring for 12 hours and the end salt is crystallized as a white microcrystalline powder. There are obtained 6 g of end product having m.p. 97/98°C.

HPLC titration:

99.16% (on dry basis)

83.3% (on the product as such).

$[\alpha]^{20}_d$ (calculated on dry basis) = - 14.45°(c = 5 $H_2O$)

EXAMPLE 3

Alpha glyceryl phosphoryl myoinositol L-lysine salt

The same reaction of example 2 is carried out using 50% L-lysine base in the same amount and according to the same process conditions leading to 6.9 g of end product with m.p. 99°C.

HPLC titration:

99.12% (on dry basis)

95.16% (on the product as such).

$[\alpha]^{20}_{d}$ (calculated on dry basis) = -8.44° (c = 5 $H_2O$).

EXAMPLE 4

Glyceryl phosphoryl mesoinositol salt with 4(1-pyrrolidinyl)1(2,4,6-trimethoxyphenyl)1-butanone

An aqueous solution of glyceryl phosphoryl mesoinositol containing 8.02 g (0.0240 moles) of active principle is added with 7.38 g of 4(1-pyrrolidinyl)1(2,4,6-trimethoxyphenyl)1-butanone as pure base. The mixture is maintained under stirring until a cpmplete aqueous solution is obtained having final pH of between 4.7 and 4.8.

This solution is subjected to rotating stirring and concentrated under vacuum until it is almost dried.It is taken with ethanol/acetone in the ratio 4/1 and then concentrated again to dryness.

A crystalline product is obtained having m.p. of 68/69°C.

HPLC titration :

102.9% (on dry basis)

84.5% (on the product as such).

$[\alpha]^{20}_{d}$ (calculated on dry basis) = - 12.66° (c = 5 $H_2O$).

## Claims

1. Saline derivatives of glyceryl phosphoryl myoinositol having the formula

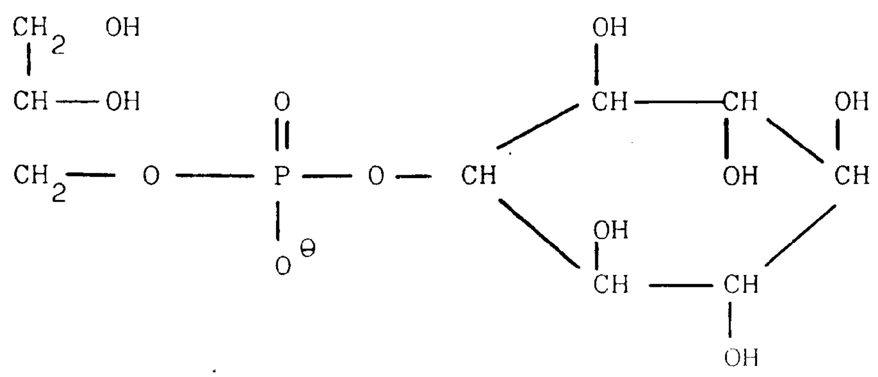

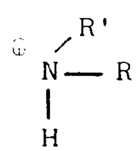

wherein R represents (a) the residue of a basic aminoacid or (b) the group of a cyclic ring having 4 to 5 carbon atoms and R′ represents hydrogen when R has the meaning (a) and a simple or substituted aliphatic chain when R has the meaning (b).

2. Saline derivatives according to claim 1, characterized in that said basic aminoacid is selected among L- and DL-lysine, arginine and ornithine.

3. Saline derivatives according to claim 1, characterized in that said group of cyclic ring is pyrrolidinyl or piperidinyl.

4. Saline derivatives according to claim 1, characterized in that R′ is the radical of 4(1-pyrrolidinyl) 1 (2,4,6-trimethoxyphenyl) 1-butanone.

**5.** A process for the preparation of saline derivatives according to claim 1, characterized in that glyceryl phosphoryl myoinositol in aqueous solution, just prepared from the corresponding cyclohexylammonium salt, is reacted with the basic aminoacid in form of basic or of aqueous solution, or with 4 (1-pyrrolidinyl) 1 (2,4,6-trimethoxyphenyl) 1-butanone, until the pH of the reaction mixture is of between 4.6 and 4.8 and then the final desired product is isolated.

**6.** Pharmaceutical composition characterized by containing as the active principle a saline derivative according to claim 1, together with the usual excipients and vehicles.

**7.** Pharmaceutical composition according to claim 6, in form suitable for oral administration.

**8.** Pharmaceutical composition according to claims 6 or 7, for the treatment of cerebral syndromes accompanying the ageing.